# Europäisches Patentamt

(19) **European Patent Office**

**Office européen des brevets**

(11) Numéro de publication: **0 152 337**
**B1**

## FASCICULE DE BREVET EUROPÉEN

(45) Date de publication du fascicule du brevet:
12.07.89

(51) Int. Cl.⁴: **A 61 K 6/06**

(21) Numéro de dépôt: **85400171.6**

(22) Date de dépôt: **01.02.85**

(54) **Procédé de fabrication d'une couche céramique basale d'opacification de la chape métallique d'une reconstitution céramo-métallique dentaire.**

(30) Priorité: **15.02.84 FR 8402295**

(43) Date de publication de la demande:
**21.08.85 Bulletin 85/34**

(45) Mention de la délivrance du brevet:
**12.07.89 Bulletin 89/28**

(84) Etats contractants désignés:
**AT BE CH DE FR GB IT LI LU NL SE**

(56) Documents cité:
**DE-A-1 441 336**
**FR-A-1 378 540**
**US-A-4 215 033**

**CHEMICAL ABSTRACTS, vol. 89, no. 22, 27 novembre 1978, page 377, no. 186102a, Columbus, Ohio, US; & SU - A - 624 622 (MOSCOW MEDICAL STOMATOLOGICAL INSTITUTE) 25-09-1978**
**PANAMAT, Mode d'Emploi, Brochure FRANCER, Suissor S.A. .**

(73) Titulaire: **SUISSOR S.A., 9, rue Franklin, F-49024 Angers (FR)**

(72) Inventeur: **Heurtaux, Michel, 48a, rue Auguste Vacher, F-41200 Romorantin (FR)**

(74) Mandataire: **Ahner, Francis, CABINET REGIMBEAU 26, avenue Kléber, F-75116 Paris (FR)**

## Description

La présente invention concerne le domaine technique des reconstitutions céramo-métalliques dentaires. Elle concerne plus particulièrement la réalisation de composition de verre céramique utilisé pour réaliser différents types de reconstitutions dentaires, tels que des couronnes, incrustations, bridges etc.

Ce type de reconstitutions dentaires est habituellement obtenu à partir d'une succession de couches de céramique venant coiffer une chape métallique par exemple déposée sur le moignon d'une dent réduite après façonnage à la fraise. Cette succession de couche céramique se compose d'une couche basale d'opacification, de deux couches intermédiaires connues sous la dénomination de "dentine" et "incisal", et d'une couche superficielle transparente destinée à rappeler l'éclat de l'émail de la dent naturelle.

L'objet de la présente invention vise plus spécialement la réalisation de la couche céramique basale d'opacification de la chape métallique d'une telle reconstitution dentaire.

Comme son nom l'indique, une telle couche est principalement destinée à dissimuler l'aspect métallique de la chape support. Il est donc essentiel de lui conférer de bonnes propriétés d'opacification. Cela n'est toutefois pas suffisant. En effet, pour obtenir une bonne résistance mécanique et une bonne ténacité à la cassure de la prothèse, il est essentiel d'assurer un parfait accrochage de la couche céramique opaque sur la chape métallique. Les diverses solutions proposées jusqu'à ce jour ne conduisent pas à une adhérence satisfaisante dans la pratique.

La présente invention a précisément eu pour but la réalisation d'une céramique dentaire opaque par application et cuisson d'au moins une fritte de verre. En plus de ses propriétés d'adhérence améliorées, cette céramique est dotée d'un excellent pouvoir opacifiant. Cette couche selon l'invention présente en outre, après cuisson, un état de surface rugueux responsable d'une bonne diffusion de la lumière, ce qui permet d'éviter tout effet réfléchissant désagréable de la prothèse qui, de ce fait, ne restituerait pas véritablement l'aspect de la dent naturelle.

Il convient en outre de noter que, du fait de son meilleur pouvoir opacifiant, la céramique dentaire selon l'invention peut être appliquée par pulvérisation, ce qui laisse plus d'espace disponible pour appliquer les couches suivantes, lesquelles deviennent ainsi plus faciles à modeler par le prothésiste.

En outre, la composition céramique dentaire opaque selon l'invention a été adaptée pour être en accord de dilatation avec ses couches adjacentes, et en particulier avec son support métallique. Cette compatibilité se trouve d'ailleurs facilitée considérablement du fait de sa meilleure opacité qui autorise son dépôt en couche plus mince.

L'idée mère, à la base de la présente invention qui permet de concilier d'une part l'adhérence et d'autre part l'opacité de ce type de couche céramique, repose sur la dissociation en deux couches distinctes de la couche opaque unique habituellement utilisée dans la technique antérieure. La première sous-couche, dite "lait d'opaque", directement au contact de l'armature, est destinée à apporter une bonne adhérence avec le métal, alors que l'autre couche dite "opaque", a été conçue pour apporter le degré d'opacité suffisant.

La présente invention concerne donc un procédé pour la réalisation d'une couche céramique basale d'opacification de la chape métallique d'une reconstitution céramo-métallique dentaire, par application et cuisson à une température comprise entre 800°C et 850°C, d'au moins une fritte, caractérisé en ce qu'on réalise une sous-couche de lait d'opaque recouverte par une couche d'opaque en appliquant par pulvérisation de, respectivement une première et une seconde fritte de verre, la première fritte présentant une granulométrie moyenne de 12 μm et étant plus riche en fondants, en particulier en $B_2O_3$, que la seconde fritte, et ladite seconde fritte présentant une granulométrie moyenne de 36 μm, étant plus riche en oxydes métalliques opacifiants tels que l'oxyde de zirconium et l'oxyde d'étain, que la première fritte, et contenant en outre 30 % en poids de grains réfractaires, lesdits grains réfractaires ayant une granulométrie comprise entre 40 μm et 100 μm.

La composition de fritte de verre utilisée dans le procédé selon l'invention peut donc être présentée telle quelle, pour être directement prête à l'emploi. Une simple pulvérisation sur la chape métallique à l'aide d'une bombe aérosol permet ainsi d'obtenir des couches minces de lait d'opaque ou d'opaque.

D'autres caractéristiques et avantages de la présente invention apparaîtront à la lecture de la description détaillée faite ci-après en s'appuyant notamment sur des exemples de mise en oeuvre particuliers, donnés à titre de simple illustration.

La fritte de verre riche en fondants, destinée à composer la sous-couche de lait d'opaque au contact de la chape métallique, contient avantageusement d'environ 8 à environ 14 % en poids et de préférence d'environ 9 à environ 11 % en poids de $B_2O_3$. C'est précisément cette teneur élevée en oxyde de bore qui permet d'améliorer l'aptitude à l'adhérence au métal de cette sous-couche de lait d'opaque. Il convient toutefois de noter que des teneurs de $B_2O_3$ supérieures à environ 14 % en poids conduiraient à des frittes de verre inutilisables dans la pratique, étant donné qu'elles deviendraient trop fusibles. En effet, une sous-couche de lait d'opaque ne peut pas présenter des températures de fusion trop basses, étant donné que sa température de fusion doit rester en tout état de cause supérieure à celles des couches supérieures suivantes qui sont déposées et thermiquement stabilisées ultérieurement.

En ce qui concerne la couche d'opaque proprement dite, elle contient bien évidemment des agents opacifiants tels que l'oxyde d'étain, l'oxyde de titane et l'oxyde de zirconium, à raison d'une teneur élevée. Toutefois, le pouvoir opacifiant de cette couche se trouve considérablement renforcé par la présence d'une proportion importante (de l'ordre de 30 % en poids) de grains réfractaires relativement gros, c'est-à-dire d'une granulométrie comprise entre environ 40 et environ 100 µm. La présence de ces grains réfractaires de dimensions relativement grandes, conduit, après cuisson, à une rugosité de la surface de cette couche d'opaque qui apporte un effet de diffusion de la lumière au travers des couches supérieures plus translucides, et améliore ainsi l'effet de "profondeur" de la dent.

On indiquera ci-après à titre d'illustration, deux exemples de composition de frittes de verre destinées à la réalisation d'une sous-couche de lait d'opaque et d'une couche d'opaque.

Exemple de composition globale de lait d'opaque:

Une telle fritte de verre riche en fondants ayant conduit à des résultats satisfaisants dans la pratique répond par exemple à la composition suivante:

$SiO_2$   46 à 48 % en poids
$Al_2O_3$ 10 à 12 % en poids
$CaO$    1 à 1,5 % en poids
$MgO$    0,5 à 1,5 % en poids
$K_2O$    10 à 11 % en poids
$Na_2O$   6 à 9 % en poids
$B_2O_3$   9 à 11 % en poids
$ZrO_2$    1,5 à 2 % en poids
$SnO_2$    6 à 8 % en poids
$TiO_2$    0,5 à 1 % en poids.

De préférence, une telle fritte de verre présente une granulométrie moyenne d'environ 12 µm.

Exemple de composition globale de couche d'opaque:

Une fritte de verre riche en oxydes métalliques opacifiants, utilisée de façon satisfaisante dans la pratique pour réaliser une couche d'opaque selon l'invention, répond par exemple à la composition globale suivante:

$SiO_2$   42 à 46 % en poids
$Al_2O_3$ 14 à 18 % en poids
$CaO$    0,8 à 1,7 % en poids
$MgO$    0,5 à 1,5 % en poids
$K_2O$    11 à 13 % en poids
$Na_2O$   3 à 6 % en poids
$B_2O_3$   5 à 8 % en poids
$ZrO_2$    3 à 5 % en poids
$SnO_2$    11 à 14 % en poids
$TiO_2$    0,5 à 3 % en poids.

La fritte de verre riche en oxydes métalliques opacifiants ci-dessus présente avantageusement une granulométrie moyenne légèrement supérieure, par exemple d'environ 36 µm, en respectant la présence d'une fraction de l'ordre de 30 % en poids de grains allant d'environ 40 à environ 100 µm, privilégiant les grains réfractaires. Cette fraction de grains réfractaires contient une proportion d'oxydes métalliques opacifiants toujours supérieure à 25 % en poids, c'est-à-dire que ces grains sont plus riches en agents opacifiants que le reste de la composition.

Les frittes de verre nécessaires à la réalisation des couches obtenues par le procédé selon la présente invention sont préparées de façon classique, à partir de mélanges pulvérulents des constituants nécessaires tels que déterminés précédemment. Un tel mélange pulvérulent est par exemple porté pendant une heure à une température de l'ordre de 1500°C, ce qui lors de la fusion conduit à une homogénéisation, suivie par exemple d'une trempe conduisant à la fritte qui peut être ultérieurement soumise à une opération de broyage ou de granulation.

Cette opération de broyage est effectuée de façon contrôlée pour atteindre les granulométries exigées conformément à la présente invention.

La présente demande se rapporte également à des frittes de verre nécessaires à la réalisation des couches céramiques dites "lait d'opaque" ou "opaque", qui sont caractérisées par une composition contenant:

10 à 30 % en poids de produits actifs,
60 à 80 % en poids d'un gaz propulseur,
10 à 30 % en poids d'un solvant ayant une température de vaporisation comprise entre environ 40 et environ 80°C,
et
4 à 6 % en poids d'une colle.

Le gaz propulseur peut être par exemple constitué par un ou plusieurs hydrocarbures chlorofluorés (Freon®). Le mélange de colle et de solvant peut par exemple être constitué par du collodion officinal dissous dans de l'acétate d'éthyle. Le solvant peut en outre être avantageusement constitué par un hydrocarbure chloré, par exemple $CH_2Cl_2$, et la colle peut également être constituée par une colle acrylique ou encore, de préférence, cyanoacrylique.

Une telle composition est ainsi apte à être conditionnée dans un flacon distributeur du type aérosol.

Ces couches sont ensuite fixées de façon classique par cuisson, au cours d'un traitement thermique à une température comprise entre 800 à 850°C, par exemple pendant 7 à 8 minutes. Il convient également de noter que du fait des compositions particulières des céramiques objet de la présente invention, il devient ainsi possible d'abaisser assez considérablement la température de cuisson pour la fixation de ces couches. En effet, dans la technique antérieure une telle température de cuisson n'était jamais inférieure à environ 950°C. Au surplus, l'ensemble de la couche basale constituée par la couche de lait d'opaque et par la couche d'opaque peut être appliqué de façon à conduire à une couche d'une épaisseur moyenne d'environ 80 µm. Dans la technique antérieure classique d'application au pinceau, il était rigoureusement impossible d'atteindre des épaisseurs inférieures à environ 120 μm.

Le traitement thermique de fixation de la couche de lait d'opaque et de la couche d'opaque nécessite en général deux cuissons successives. Toutefois, lorsque les frittes de verre nécessaires à la réalisation de couches de lait d'opaque et d'opaque sont déposées à l'aide d'une composition aérosol contenant par exemple une colle cyanoacrylique, il est possible de fixer l'ensemble des couches d'opaque et des couches successives au cours d'une et même cuisson. Immédiatement après la projection, le gaz propulseur et les solvants s'éliminent rapidement, et la colle remplit son rôle de fixateur des couches sur l'armature. Le solvant ou le plastifiant projeté lors du dépôt des couches successives, de dentine, d'incisal et de transparent, est choisi de manière à ne pas dissoudre un tel type de colle. En conséquence, les couches basales ne se trouvent pas affectées par le dépôt des couches suivantes, et il est ainsi possible de fixer l'ensemble des couches céramiques au cours d'une seule et même cuisson, ce qui représente un gain de temps considérable. On notera également que les plus faibles températures de cuisson des couches basales se rapprochent ainsi de celles des autres couches et permettent précisément d'envisager une mono-cuisson de l'ensemble de la prothèse céramo-métallique.

**Revendications**

1. Procédé pour la réalisation d'une couche céramique basale d'opacification de la chape métallique d'une reconstitution céramo-métallique dentaire, par application et cuisson à une température comprise entre 800°C et 850°C d'au moins une fritte, caractérisé en ce qu'on réalise une sous-couche de lait d'opaque recouverte par une couche d'opaque en appliquant par pulvérisation de, respectivement une première et une seconde fritte de verre, la première fritte présentant une granulométrie moyenne de 12 μm et étant plus riche en fondants, en particulier en $B_2O_3$, que la seconde fritte, et ladite seconde fritte présentant une granulométrie moyenne de 36 μm, étant plus riche en oxydes métalliques opacifiants tels que l'oxyde de zirconium et l'oxyde d'étain, que la première fritte, et contenant en outre 30 % en poids de grains réfractaires, lesdits grains réfractaires ayant une granulométrie comprise entre 40 μm et 100 μm.

2. Procédé selon la revendication 1, caractérisé en ce que la fritte de verre nécessaire à la réalisation de la sous-couche de lait d'opaque répond à la composition suivante:

$SiO_2$  46 à 48 % en poids
$Al_2O_3$  10 à 12 % en poids

$CaO$  1,0 à 1,5 % en poids
$MgO$  0,5 à 1,5 % en poids
$K_2O$  10 à 11 % en poids
$Na_2O$  6 à 9 % en poids
$B_2O_3$  9 à 11 % en poids
$ZrO_2$  1,5 à 2 % en poids
$SnO_2$  6 à 8 % en poids
$TiO_2$  0,5 à 1 % en poids.

3. Procédé selon la revendication 1, caractérisé en ce que la fritte de verre nécessaire à la réalisation de la couche d'opaque répond à la composition suivante:

$SiO_2$  42 à 46 % en poids
$Al_2O_3$  14 à 18 % en poids
$CaO$  0,8 à 1,7 % en poids
$MgO$  0,5 à 1,5 % en poids
$K_2O$  11 à 13 % en poids
$Na_2O$  3 à 6 % en poids
$B_2O_3$  5 à 8 % en poids
$ZrO_2$  3 à 5 % en poids
$SnO_2$  11 à 14 % en poids
$TiO_2$  0,5 à 3 % en poids.

4. Procédé selon l'une quelconque des revendications 1 à 3, caractérisé en ce que chaque fritte de verre nécessaire à la réalisation desdites sous-couche de lait d'opaque et couche d'opaque, est présentée sous la forme d'une composition contenant:

10 à 30 % en poids de produits actifs,
60 à 80 % en poids d'un gaz propulseur,
10 à 30 en poids d'un solvant ayant une température de vaporisation comprise entre environ 40 et 80°C, et
4 à 6 en poids d'une colle.

5. Procédé selon la revendication 4, caractérisé en ce que chaque fritte de verre est présentée sous la forme d'une composition contenant:

10 à 30 % en poids de produits actifs,
60 à 80 % en poids d'un gaz propulseur constitué par un ou plusieurs hydrocarbures chlorofluorés,
10 à 30 % en poids de $CH_2Cl_2$
4 à 6 % en poids d'une colle cyanoacrylique.

**Patentansprüche**

1. Verfahren zum Ausbilden einer keramischen Grundtrübungsschicht für die Metallkrone eines keramisch-metallischen Zahnwiederaufbaus durch Aussetzen und Aushärten wenigstens einer Fritte bei einer Temperatur zwischen 800°C und 850°C, dadurch gekennzeichnet, daß eine milchigtrübe Unterschicht ausgebildet wird, die von einer undurchsichtigen Schicht überzogen ist, indem über eine Pulverisierung jeweils eine erste und eine zweite Glasfritte aufgebracht werden, wobei die erste Fritte eine mittlere Korngröße von 12 μm hat und reicher an Flußmitteln, ins-

besondere $B_2O_3$ als die zweite Fritte ist, und wobei die zweite Fritte eine mittlere Korngröße von 36 µm hat, reicher an undurchsichtigen Metalloxiden wie Zirkonoxid und Zinnoxid als die erste Fritte ist und außerdem 30 Gewichtsprozent hochschmelzende Körner enthält, wobei die hochschmelzenden Körner eine Korngröße zwischen 40 µm und 100 µm haben.

2. Verfahren nach Anspruch 1, dadurch <u>gekennzeichnet</u>, daß die Glasfritte, die zum Ausbilden der milchigtrüben Unterschicht benötigt wird, die folgende Zusammensetzung hat:

$SiO_2$ 46 bis 48 Gewichtsprozent
$Al_2O_3$ 10 bis 12 Gewichtsprozent
CaO 1,0 bis 1,5 Gewichtsprozent
MgO 0,5 bis 1,5 Gewichtsprozent
$K_2O$ 10 bis 11 Gewichtsprozent
$Na_2O$ 6 bis 9 Gewichtsprozent
$B_2O_3$ 9 bis 11 Gewichtsprozent
$ZrO_2$ 1,5 bis 2 Gewichtsprozent
$SnO_2$ 6 bis 8 Gewichtsprozent
$TiO_2$ 0,5 bis 1 Gewichtsprozent.

3. Verfahren nach Anspruch 1, dadurch <u>gekennzeichnet</u>, daß die Glasfritte, die zur Ausbildung der undurchsichtigen Schicht benötigt wird, die folgende Zusammensetzung hat:

$SiO_2$ 42 bis 46 Gewichtsprozent
$Al_2O_3$ 14 bis 18 Gewichtsprozent
CaO 0,8 bis 1,7 Gewichtsprozent
MgO 0,5 bis 1,5 Gewichtsprozent
$K_2O$ 11 bis 13 Gewichtsprozent
$Na_2O$ 3 bis 6 Gewichtsprozent
$B_2O_3$ 5 bis 8 Gewichtsprozent
$ZrO_2$ 3 bis 5 Gewichtsprozent
$SnO_2$ 11 bis 14 Gewichtsprozent
$TiO_2$ 0,5 bis 3 Gewichtsprozent.

4. Verfahren nach einem der Ansprüche 1 bis 3, dadurch <u>gekennzeichnet</u>, daß jede Glasfritte, die zum Ausbilden der milchigtrüben Unterschicht und der undurchsichtigen Schicht benötigt wird, in Form eines Gemisches vorliegt, das 10 bis 30 Gewichtsprozent aktive Produkte, 60 bis 80 Gewichtsprozent Treibgas, 10 bis 30 Gewichtsprozent eines Lösungsmittels mit einer Verdampfungstemperatur zwischen etwa 40 und 80°C und 4 bis 6 Gewichtsprozent eines Klebstoffes enthält.

5. Verfahren nach Anspruch 4, dadurch <u>gekennzeichnet</u>, daß jede Glasfritte die Form eines Gemisches hat, das 10 bis 30 Gewichtsprozent aktive Produkte, 60 bis 80 Gewichtsprozent eines Treibgases, das von einem oder mehreren Fluorchlorkohlenwasserstoffen gebildet wird, 10 bis 30 Gewichtsprozent $CH_2Cl_2$ und 4 bis 6 Gewichtsprozent eines Zyanacrylklebstoffes enthält.

## Claims

1. Process for producing a ceramic basal layer for opacifying the metal coping of a ceramo-metallic dental reconstruction by the application and firing at a temperature of between 800°C and 850°C of at least one frit, characterized in that an undercoat of opaque slip covered with an opaque layer is produced by applying, by spraying, a first and second glass frit respectively, the first frit having a mean particle size of 12 µm and being richer in fluxes, particularly in $B_2O_3$, than the second frit, and the said second frit having a mean particle size of 36 µm, being richer in opacifying metal oxides, such as zirconium oxide and tin oxide, than the first frit and additionally containing 30 % by weight of refractory particles, the said refractory particles having a particle size of between 40 µm and 100 µm.

2. Process according to Claim 1, characterized in that the glass frit required for producing the undercoat of opaque slip corresponds to the following composition:

$SiO_2$ 46 to 48 % by weight
$Al_2O_3$ 10 to 12 % by weight
CaO 1.0 to 1.5 % by weight
MgO 0.5 to 1.5 % by weight
$K_2O$ 10 to 11 % by weight
$Na_2O$ 6 to 9 % by weight
$B_2O_3$ 9 to 11 % by weight
$ZrO_2$ 1.5 to 2 % by weight
$SnO_2$ 6 to 8 % by weight
$TiO_2$ .5 to 1 % by weight.

3. Process according to Claim 1, characterized in that the glass frit required for producing the opaque layer corresponds to the following composition:

$SiO_2$ 42 to 46 % by weight
$Al_2O_3$ 14 to 18 % by weight
CaO 0.8 to 1.7 % by weight
MgO 0.5 to 1.5 % by weight
$K_2O$ 11 to 13 % by weight
$Na_2O$ 3 to 6 % by weight
$B_2O_3$ 5 to 8 % by weight
$ZrO_2$ 3 to 5 % by weight
$SnO_2$ 11 to 14 % by weight
$TiO_2$ 0.5 to 3 % by weight.

4. Process according to any one of Claims 1 to 3, characterized in that each glass frit required for producing the said undercoat of opaque slip and the said opaque layer, is presented in the form of a composition containing:

10 to 30 % by weight of active products,
60 to 80 % by weight of a propellent gas,
10 to 30 % by weight of a solvent with a vaporization temperature between approximately 40 and 80°C, and
4 to 6 % by weight of an adhesive.

5. Process according to Claim 4, characterized in that each glass frit is presented in the form of a composition containing:

10 to 30 % by weight of active products,
60 to 80 % by weight of a propellent gas consisting of one or more chlorofluorinated hydrocarbons,
10 to 30 % by weight of $CH_2Cl_2$
4 to 6 % by weight of a cyanoacrylic adhesive.